# FASCICULE DE BREVET EUROPEEN

(11) **EP 1 232 188 B1**
(45) Date de publication et mention de la délivrance du brevet: **27.10.2004**
(21) Numéro de dépôt: 00956571.4
(22) Date de dépôt: 28.07.2000
(51) Int. Cl.: C08B 37/16, A61K 9/10, A61K 9/14

(54) **CYCLODEXTRINES SUBSTITUEES PAR DES GROUPEMENTS FLUOROALKYLES, LEUR PREPARATION ET LEUR UTILISATION**
CYCLODEXTRINE, DIE DURCH FLUOROALKYLGRUPPEN SUBSTITUIERT SIND, DEREN HERSTELLUNG UND VERWENDUNG
CYCLODEXTRINS SUBSTITUTED BY FLUOROALKYL GROUPS, PREPARATION AND USE THEREOF

(30) Priorité: 29.07.1999 FR 9909863
(43) Date de publication de la demande: 21.08.2002
(73) Titulaire: UNIVERSITE DE ROUEN, 76821 Mont-Saint-Aignan Cédex (FR)
(72) Inventeur: SKIBA, Mohamed, F-76000 Rouen (FR); SKIBA, Malika, F-76000 Rouen (FR); DUCLOS, Roselyne, F-76240 Bonsecours (FR); COMBRET, Jean-Claude, F-76000 Rouen (FR); ARNAUD, Philippe, F-76000 Rouen (FR)
(74) Mandataire: Breese, Pierre
(86) Numéro de dépôt international: PCT/FR2000/002192
(87) Numéro de publication internationale: WO 2001/009195

(56) Documents cités:
- JP-A- 9 241 303
- US-A- 5 739 121

## Description

Les cyclodexrines sont de oligomères cycliques bâtis à partir d'unités alpha-D-glucopyranosyl en conformation chaise reliés par des liaisons 1-4, ci-après aussi désignés par résidu(s) cyclodextrinyle(s).

Les cyclodextrines peuvent être représentées par la formule générale I suivante :

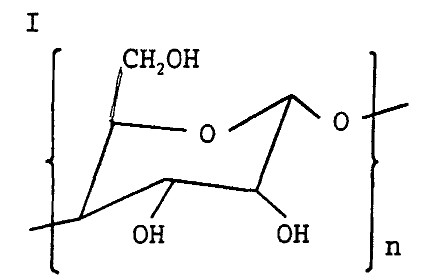

Les alpha, bêta et gamma cyclodextrines sont constitués de 6, 7 et 8 de ces unités, ce qui correspond dans la formule I à n égal à 6, 7 ou 8 :
- les cyclodextrines alpha comprennent 6 groupes hydroxyles primaires en position 1, et 12 groupes hydroxyles secondaires en position 2 et 3;
- les cyclodextrines bêta comprennent 7 groupes hydroxyles primaires en position 1, et 14 groupes hydroxyles secondaires en position 2 et 3;
- les cyclodextrines gamma comprennent 8 groupes hydroxyles primaires en position 1, et 24 groupes hydroxyles secondaires en position 2 et 3;

Les cyclodextrines présentent une forme spatiale en tronc de cône. Les fonctions alcools primaires sont sur la petite base et les fonctions alcools secondaires sur la grande base, ce qui localise le caractère hydrophile à l'extérieur des cyclodextrines. La cavité interne est tapissée par des liaisons hydrogènes liées aux carbones 3 et 5 et aux atomes d'oxygène glycosidiques, ce qui confère un caractère relativement hydrophobe à la cavité des cyclodextrines.

Les cyclodextrines alpha, bêta, gamma, et de nombreux dérivées de celles-ci sont décrits depuis longtemps en tant que molécules capables de piéger certaines petites molécules de nature hydrophobe ou hydrophile. En particulier, l'utilisation de cyclodextrines pour capter et protéger les vitamines lipophiles, les arômes et certains principes actifs très fragiles a fait l'objet de nombreux brevets et publications. Ainsi, le brevet japonais JP 52/130904 rapporte la stabilisation de la vitamine D3 par inclusion dans la béta-cyclodextrine, le brevet JP 56/139409 décrit l'inclusion de la vitamine E dans les cyclodextrines, le brevet belge BE 888736 concerne la stabilisation d'un médicament, l'indométacine dans un dérivé méthylé de la β-cyclodextrine.

Cependant, l'utilisation industrielle de ces complexes cyclodextrine/actif pose plusieurs difficultés liées notamment à leur dégradation rapide dans les milieux biologiques, et à la faible interaction des cyclodextrines les membranes biologiques à cause de leur hydrophilie.

Le document US-A-5 739 121 décrit des dérivés de cyclodextrine fluorés et leur complexes d'inclusion avec des substances médicamenteuses. Le document JP-A-9 241 303 divulgue des dérivés fluorés de cylodextrine possédant une chaîne perfluorooxyalkyl utilisés comme agents modificateurs de surface dans le domaine des bandes magnétiques le bras de liaison X pouvant être choisi parmi -O-CO- ou -NH-CO- et le substituant RF étant une chaîne perfluorooxyalkyl ou perfluoroalkyl.

Les travaux de recherche réalisés par les Inventeurs sur des dérivés de cyclodextrines, leur ont permis de mettre en évidence que certains dérivés fluorés des cyclodextrines possèdent des propriétés amphiphiles qui leur permettent d'être incorporés indifféremment dans des milieux aqueux ou des milieux lipidiques, voire même des environnements amphiphiles tels les membranes des liposomes. Plus particulièrement, la présence de chaînes fluoroalkyles sur ces dérivés fluorés des cyclodextrines augmente de façon remarquable leur demi-vie dans les milieux biologiques, permettant leur utilisation beaucoup plus souple et étend le champ d'application des cyclodextrines en tant que molécules cages.

Les dérivés de cyclodextrines selon l'invention sont des cyclodextrines monosubstitués à persubstitués sur les groupes hydroxyles en position 2 et/ou 3 (sites secondaires) par un ou plusieurs groupements fluoroalkyles identiques ou différents. Ces dérivés de cyclodextrines selon l'invention sont constitués de 6 à 8 résidus cyclodextrinyles identiques ou différents de formule IV suivante : 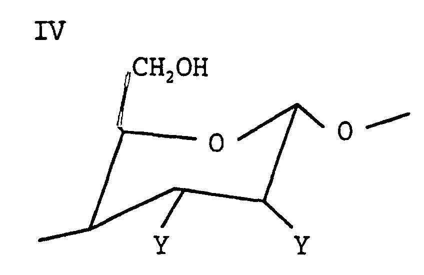 où l'un au moins des groupes Y de l'un au moins des 6 à 8 résidus cyclodextrinyles représente un groupement de formule -(X)-RF, et Y lorsqu'il n'est pas un groupement de formule -(X)-RF est un groupe -OH, où RF et X sont comme définis dans la formule II ci-dessus.

Dans cette classe de dérivés selon l'invention, les dérivés de cyclodextrines alpha comprennent de 1 à 12 radicaux RF, les dérivés de cyclodextrines bêta comprennent de 1 à 14 radicaux RF, les dérivés de cyclodextrines gamma comprennent de 1 à 16 radicaux RF.

Parmi les dérivés de cyclodextrine appartenant à cette classe, on préfère tout particulièrement ceux dans lesquels X est -CO-O ou -O-CO-, et RF est de C₃F₇ à C₁₄F₂₉.

Les dérivés de cyclodextrines selon l'invention peuvent être préparés de la façon suivante: on procède à une étape de protection des OH primaires par le chlorosilane puis on effectue une perfluoration des OH secondaire, et l'on déprotège les OH primaires.

A titre d'exemple d'un procédé de préparation des dérivés de l'invention on trouvera dans la partie expérimentale ci-après la synthèse détaillée d'une β-cylodextrine per 2,3 di-O-pentadecafluoro-octanoyle.

Les dérivés de cyclodextrines selon l'invention sont remarquables en ce qu'ils ont permis aux Inventeurs de mettre au point un nouveau système de transport de substances actives très diverses. En effet, les cyclodextrines perfluorés selon l'invention présentent de nombreux avantages par rapport notamment aux dérivés acryliques utilisés pour la préparation de particules submicroniques contenant une molécule active. Leur principal avantage réside dans le fait que, contrairement aux autres dérivés acryliques dont la polymérisation requiert un apport énergétique susceptible de nuire à la stabilité du principe actif incorporé, les cyclodextrines perfluorées ne se polymérisent pas pour former les systèmes transporteurs.

En outre, les dérivés de cyclodextrines selon l'invention autorisent un taux d'incorporation d'une substance active très élevé. Ceci est dû à la possibilité de triple charge, en premier lieu, une charge dans le réseau, en second lieu dans la cavité de la cyclodextrine à condition que la molécule encapsulée ait une conformation convenable par rapport à la cavité, et en troisième lieu une charge adsorbée à la surface de la particule formée par le dérivé de cyclodextrine de l'invention.

Enfin, les cyclodextrines perfluorées selon l'invention possèdent l'avantage de solubiliser les gaz.

Les systèmes de transport mis au point par les inventeurs à partir des dérivés de cyclodextrine selon l'invention trouvent des applications dans de nombreux secteurs techniques.

En effet, les dérivés de cyclodextrine selon l'invention permettent de préparer des particules capables de transporter ou de vectoriser des substances très diverses, tels que de l'oxygène ou d'autre gaz, du fait de la grande capacité des fluorures de dissoudre les gaz, et en conséquence présentent de nombreuses applications comme par exemple substituts temporaires ou autres du sang, hemodilution préopératoire, traitement des ischémies myocardites et cérébrales, cardioplegie, perfusion, potentialisation de la radio et de la chimiothérapie du cancer, préservations d'organes et diagnostic.

Les particules de l'invention permettent de vectoriser des principes actifs utiles en thérapeutique humaine et animale. Plus particulièrement leur utilisation permet :
- d'atteindre de nouveaux sites d'action, en particulier intracellulaires, voire intralysomiaux,
- de mettre en oeuvre de nouvelles voies d'administration en augmentant la stabilité et/ou l'absorption des principes actifs, ou en permettant la réalisation de formes injectables par voie intravasculaire, de principes actifs insolubles
- de modifier la distribution tissulaire des principes actifs, par un meilleur ciblage vers des sites d'actions favorables et/ou un détournement des sites d'effets indésirables voire toxiques (amélioration de l'index thérapeutique).

Tout particulièrement, les dérivés de cyclodextrine de l'invention permettent de préparer des dispersions colloïdales utiles pour réaliser des formes injectables de médicaments insolubles, ou stabiliser un principe actif médicamenteux.

Les particules de l'invention trouve également des applications dans des domaines autres que ceux de la médecine, comme par exemple en phytopharmacie, où elles permettent de véhiculer des insecticides ou des pesticides. Du fait de leur taille, les particules de l'invention pénétrent facilement à travers la cuticule. La faible viscosité de la dispersion autorise une pulvérisation très facile sous forme de gouttelettes de très petite taille plus efficaces car plus couvrantes.

Dans le domaine cosmétique et dermatologique, les particules de l'invention peuvent transporter des substances anti-radicalaires ou de l'oxygène au niveau du derme.

Les particules de l'invention trouvent également des applications dans le domaine des cultures aérobies, de la fermentation et de la conservation d'organes, en raison des propriétés de transport en matrice liquide inerte de gaz réactifs (O₂, hydrogène, oxyde de carbone ou dioxyde) ou non (azote ou gaz rares).

Dans des domaines industriels, comme les peintures, vernis et traitements des surfaces d'une manière générale, les particules de l'invention permettent de disposer de dispersions utiles pour véhiculer des pigments, des réactifs, des décapants sous forme de dispersion aqueuse de très faible viscosité, aisées à pulvériser ou à appliquer, et qui peuvent, si nécessaire, être rendues visqueuses, voire adhésives (remise en suspension des particles dans un véhicule approprié). La taille réduite des particules conduit à une très grande finesse du dépôt et à une très grande homogénéité, par exemple de pigmentation permettant leur utilisation dans l'imprimerie, la reprographie, le traitement de surface des textiles et des fibres, dans la photographie, la lubrification, l'agriculture.

L'invention se rapporte donc également à des nanoparticules de taille inférieure à environ 1000 nm constituées de dérivés de cyclodextrine décrits précédemment et éventuellement d'une ou plusieurs substances actives.

Les dérivés de cyclodextrines utilisés pour la fabrication de nanoparticules de taille inférieure à 1000 nm qui encapsulent une ou plusieurs substances active(s) selon l'invention sont constitués de 6 à 8 résidus cyclodextrinyles identiques ou différents de formule II suivante : 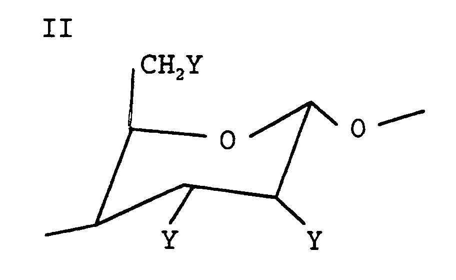 où l'un au moins des groupes Y de l'un au moins des 6 à 8 résidus cyclodextrinyles représente un groupement de formule -(X)-RF, et Y lorsqu'il n'est pas un groupement de formule -(X)-RF est un groupe -OH, où RF est un radical fluroalkyl de 1 à 20 carbones ou plus dont la chaîne carbonée est éventuellement interrompue par un ou plusieurs hétéroatomes comme l'oxygène ou le soufre, linéaire ou ramifié par un groupe alkyle ou fluroalkyl saturée ou non de 1 à 20 carbones ou plus, neutre, zwitterionique, chargée fonctionnalisée par exemple par un ou plusieurs groupes - COOH, -OH, CF3, et X représente un bras de liaison entre un atome de carbone et le groupement de formule RF.

Un radical fluroalkyl RF contenant plus de 20 atomes de carbone entre également dans le cadre de la présente invention. De même, le radical fluroalkyl RF peut être linéaire ou ramifié par une chaîne alkyle ou fluroalkyl saturée ou non comprenant de 1 à 20 carbones ou plus, neutre, zwitterionique, chargée fonctionnalisée ou sein de la chaîne ou à l'une au moins de ses extrémités. A titre d'exemple d'une chaîne fluoroalkyle selon l'invention, on peut citer le groupe suivant : -CF₂-CF₂-O-CF₂-CF₂-O-CF₃ .

Le bras de liaison X peut être une liaison covalente ou un atome ou un groupe liant l'atome de carbone du résidu cyclodextrinyle et le radical fluroalkyl RF, comme par exemple : -NH-, -CO-O-, -O-CO-, -CO-NH-, -NH-CO-, -O-, -O-(CH₂)ₙ-, -(CH₂)ₙ-CO-O-, -O-CO-NH-, PO₄⁻ ou S.

On préfère parmi les dérivés de cyclodextrine selon l'invention, ceux dans lesquels :
- RF représente de CF₃ à C₁₄F₂₉,
- X représente -CO-O-, -CO-, amine et amide.

Selon une première forme de réalisation, les nanoparticules de l'invention sont sphériques de type vésiculaire. Ces nanoparticules ont permis de préparer un nouveau système colloïdal nanovésiculaire dispersible.

Selon les conditions opératoires, il est possible d'obtenir des nanocapsules sphériques de type vésiculaire dont le diamètre varie de quelques dizaines à plusieurs centaines de nanomètres. La préparation des nanocapsules présente l'avantage d'être réversible. IL est possible de solubiliser les nanocapsules et de les préparer à partir de cette solution suivant le mode opératoire.

Un procédé de préparation de nanoparticules de type vésiculaire selon l'invention comprend les étapes suivantes :
i) on prépare une phase liquide constituée essentiellement par une solution de cyclodextrine modifiée et d'une huile dans un solvant ou mélange de solvants contenant ou non un surfactif et pouvant être additionnée d'une molécule active.
ii) on prépare une seconde phase liquide constituée essentiellement par de l'eau ou d'un mélange aqueux, contenant ou non un surfactif et pouvant être additionnée d'une molécule active.
iii) on ajoute sans agitation modérée, l'une des phases liquides obtenues sous (i) ou (ii) à l'autre, de manière à obtenir pratiquement instantanément une suspension colloïdale de nanocapsules de cyclodextrine modifiée.
iv) éventuellement, on élimine tout ou une partie du solvant ou du mélange de solvants et du non-solvant ou du mélange de non-solvants, de manière à obtenir une suspension colloïdale de concentration voulue en nanocapsules ou à obtenir une poudre de nanocapsules.

La cyclodextrine modifiée est notamment une cyclodextrine naturelle ou modifiée, modifiée par voie chimique, biochimique, enzymatique ou autre, par addition de chaîne aliphatique pouvant contenir un ou plusieurs groupements fonctionnels, aux niveaux des sites de carbones primaires ou secondaires, telle qu'une béta-cyclodextrine avec 3 carbones, 12 carbones, 14 carbones ou plus ou moins. La molécule active peut être un principe médicamentaux ou un précurseur médicamenteux, un réactif biologique ou un principe cosmétique. L'invention permet d'obtenir des nanocapsules de cyclodextrine modifiée seules (utilisables telles quelles) ou avec la molécule active.

Le solvant dans la phase (i) qui est l'acétone ou un mélange de solvants se trouve notamment à une température allant de 0° à 50°C par exemple environ à la température ambiante.

L'huile peut être une huile végétale ou minérale, ou toute substance huileuse, par exemple l'huile d'olive, le benzoate de benzyle, le myristate d'isopropyle, des glycérides d'acide gras (p.ex. un Miglyol®), ou autre.

Le non-solvant dans la phase (ii), qui est de l'eau ou un mélange aqueux, se trouve notamment à une température allant de 0° à 100°C ou plus (sous pression atmosphérique), par exemple environ à la température d'ébullition.

La concentration de cyclodextrine modifiée dans la phase (i) peut varier.

Le rapport des volumes phase (i)/phase (ii) peut varier de 0,1 à 1, préférentiellement de 0,2 à 0,6.

Finalement, la suspension colloïdale de nanocapsules peut être à volonté concentrée, stérilisée, tamponnée (par exemple au pH physiologique), lyophilisée.

L'invention permet d'obtenir des nanocapsules de cyclodextrines perfluorées notamment de 150 à 300nm.

Ces nanoparticules sont susceptibles de contenir un ou plusieurs principes actifs. On entend par principe actif plus particulièrement des agents pharmaceutiques, cosmétiques, dermatologiques ou même alimentaires, mais il peut aussi s'agir de substances actives diverses utiles pour les multiples applications données précédemment.

Le taux d'encapsulation est toujours très élevé dans le cas de produits lipophiles; 200 mg de cyclodextrine modifiée permet par exemple d'encapsuler 3 ml d'huile de benzoate de benzyle ou 230 mg de progestérone et 0,6 ml d'huile utilisée comme support.

Les substances actives insolubles dans les solvants organiques, et solubles dans l'eau peuvent également être encapsulées. Il suffit qu'elles présentent une certaine affinité pour la phase lipophile; le procédé de préparation des nanocapsules est analogue. Il suffit de solubiliser la substance active dans la phase aqueuse contenant les dérivés de cyclodextrines. Le taux d'encapsulation dépend du coefficient du partage de la substance active et de sa lipophilie.

Les nanocapsules de l'invention, contenant une huile, sont stables à l'autoclave pendant plus de 15 mn à 120°C sous une pression d'1 bar.

Les nanocapsules de l'invention sont stables à l'ultracentrifugation. Après une ultracentrifugation à 220 000 g pendant 2 h 30 mn, la plus grande part des nanocapsules peut être facilement redispersée dans l'eau.

Les résultats précédents ne peuvent s'expliquer que par la présence d'une paroi enveloppant le matériel encapsulé et que par encapsulation complète du principe actif.

La substance active, contenue dans les nanocapsules de l'invention, est plus particulièrement une molécule médicamenteuse à usage humain ou vétérinaire ou un produit pour le diagnostic. Comme molécule médicamenteuse, on peut citer plus particulièrement les produits chimiques doués de propriétés pharmacologiques et par exemple, les substances antimitotiques ou antinéoplasiques comme le méthotréxate, l'actinomycine D, l'adriamycine, la daunorubicine, la bléomycine, et la vincristine ou les substances antibiotiques comme les pénicillines, les céphalosporines et l'acide nalidixique, les antibiotiques du type aminoglycoside et ceux de la famille de la virginiamycine et les substances hormonales, notamment les hormones stéroïdiennes. Ces molécules médicamenteuses peuvent être notamment des composés chimiques à haut poids moléculaire comme l'insuline et l'héparine. Il peut s'agir également des produits biologiques comme des antigènes, des enzymes, des protéines, des virus ou des constituants de virus, de bactéries ou de cellules. Les nanoparticules suivant l'invention peuvent également contenir un produit pour le diagnostic comme par exemple la fluorescéine et la séralbumine humaine radio-active. Les nanoparticules suivant l'invention peuvent également contenir un produit pour le diagnostic, notamment des produits radio-opaques lipophiles tels que les huiles iodées.

En médecine humaine ou vétérinaire, les nanoparticules de l'invention peuvent être administrées avec ou sans excipient adéquat par voie orale, sous cutanée, intradermique, intramusculaire ou intraveineuse et leur diffusion dans les tissus les rend particulièrement intéressantes pour les traitements par voie générale.

Selon une seconde forme de réalisation, les nanoparticules de l'invention sont sphériques de type matriciel. Ces nanoparticules, comme les précédentes, ont une taille inférieure à 1000 nm et seront donc aussi désignées nanosphères. Elles contiennent ou non une ou plusieurs substances actives.

Ces particules sphériques de type matriciel constituent un système transporteur dont la préparation a l'avantage d'être réversible. Il est en effet possible de solubiliser les systèmes transporteurs et préparer les systèmes transporteurs à partir de cette solution suivant le mode opératoire. Un procédé de préparation préféré de ces particules sphériques de type matriciel comprend les étapes suivantes :
a) on prépare une phase liquide constituée essentiellement par une solution de dérivés de cyclodextrine selon l'invention dans un solvant ou mélange de solvants contenant ou non un surfactif et pouvant être additionnée d'une molécule active,
b) on prépare une seconde phase liquide constituée essentiellement par de l'eau ou d'un mélange aqueux, contenant ou non un surfactif et pouvant être additionnée d'une molécule active,
c) on ajoute sous agitation modérée, l'une des phases liquides préparés aux étapes précédentes à l'autre, de manière à obtenir pratiquement instantanément une suspension colloïdale,
d) éventuellement, on élimine tout ou une partie du solvant ou du mélange de solvants et du non-solvant ou du mélange de non-solvants, de manière à obtenir une suspension colloïdale de concentration voulue ou une poudre.

Le solvant dans la phase de l'étape (a) qui est l'acétone ou un mélange de solvants se trouve avantageusement à une température allant de 0° à 50°C par exemple environ à la température ambiante.

Le non-solvant dans la phase de l'étape (b) qui est de l'eau ou un mélange aqueux, se trouve avantageusement à une température allant de 0° à 100°C ou plus (sous pression atmosphérique), par exemple environ à la température d'ébullition.

La concentration de dérivés de cyclodextrine dans la phase de l'étape (a) peut varier.

Le rapport des volumes de la phase de l'étape (a) / la phase de l'étape (b) peut varier de 0,1 à 1, préférentiellement de 0,2 à 0,6.

L'invention concerne donc aussi une suspension colloïdale des nanoparticules sphériques de type matriciel. Celle-ci constitue un système transporteur des dérivés de cyclodextrine perfluoré seuls ou associés à un ou plusieurs agent actif. Cette suspension colloïdale peut être à volonté concentrée, stérilisée, tamponnée (par exemple au pH physiologique), lyophilisée. Les nanoparticules de l'invention dans cette suspension colloïdale ont une taille de l'ordre de 150 à 300 nm.

La nature des principes actifs incorporés dans les nanoparticules sphériques de type matriciel et leur mode d'administration sont identiques à ceux décrits précédemment pour les nanoparticules sphérique de type vésiculaire. Plus particulièrement, les nanoparticules sphériques de type matriciel de l'invention contiennent au sein de leur réseau une molécule active par exemple une molécule médicamenteuse à usage humain ou vétérinaire ou un produit pour le diagnostic. Comme molécule médicamenteuse, on peut citer plus particulièrement les produits chimiques doués de propriétés pharmacologiques et par exemple, les substances antimitotiques ou antinéoplasiques comme le méthotréxate, l'actinomycine D, l'adriamycine, la daunorubicine, la bléomycine, et la vincristine ou les substances antibiotiques comme les pénicillines, les céphalosporines et l'acide nalidixique, les antibiotiques du type aminoglycoside et ceux de la famille de la virginiamycine et les substances hormonales, notamment les hormones stéroïdiennes. Ces molécules médicamenteuses peuvent être notamment des composés chimiques à haut poids moléculaire comme l'insuline et l'héparine et l'expression "molécule médicamenteuse" comprend également des produits biologiques comme les antigènes, les enzymes, les protéines, les virus ou des constituants de virus, de bactéries ou de cellules. Les systèmes transporteurs suivant l'invention peuvent également contenir un produit pour le diagnostic comme par exemple la fluorescéine et la séralbumine humaine radioactive.

D'autres avantages et caractéristiques de l'invention apparaîtront de la description qui suit concernant des exemples de préparation de dérivés de cyclodextrines selon l'invention et leur utilisation pour la fabrication de particules sphériques de type vésiculaire ou matriciel de taille inférieure à 1000 nm et contenant ou non une molécule active.

### Exemple 1 : Synthèse d'une β-cylodextrine per 2,3 di-O-pentadecafluoro-octanoyle, per 6-O-terbutyldiméthylsilyle.

### 1) Synthèse de l'heptakis-6-O-tert-butyldiméthylsilyl-β-cyclodextrine.

### a) Équation - bilan.

6g β-CD dans 150 ml pyridine + 3 g imidazole + 6 g ClTBDMS (léger excès) dans 50 ml pyridine.

### b) Précautions.

- La pyridine est distillée sur CaH₂
- La β-CD est séchée par entraînement azéotropique à la pyridine ou par chauffage prolongé en étuve à vide.
- .Le chlorosilane et l'imidazole sont les produits commerciaux purs (Acros Organics France ou Sigma-Aldrich).

### c) Montage.

Tricol à fond plat de 500ml équipé d'une ampoule de coulée, d'une garde desséchante, d'un septum avec une arrivée d'azote. Agitation magnétique avec barreau aimanté.

### d) Mode opératoire.

A la βCD en solution dans la pyridine, on ajoute l'imidazole sous agitation jusqu'à la mise en solution complète.

Le chlorosilane en solution dans 45ml de pyridine est alors ajouté goutte à goutte à température ambiante sous atmosphère d'azote et l'agitation est maintenue pendant 4 heures./

Le milieu réactionnel est alors jeté dans 400ml d'eau glacée puis filtré et lavé avec le minimum d'eau (50ml).

Le solide ainsi isolé est repris dans 100ml de CHCl₃ et lavé avec 50ml d'HCl à 3%, 50ml de NaHCO₃ saturé et 50ml d'eau.

La phase organique ainsi traitée est séchée sur Na₂SO₄, filtrée et évaporée.

Le solide blanc, poudreux ainsi obtenu est formé avec un rendement moyen de 85-90%.

### 2) Synthèse du réactif fluoré.

Il s'agit ici d'un des moyens d'activation retenus pour une introduction mieux maîtrisée du motif perfluoré à partir de l'acide commercial correspondant.

### a) Équation - bilan.

1g carbonyl d'imidazole dans 5 ml THF + 5,11 g acide perfluoré (2 éq.) dans 4 ml THF.

### b) Précautions.

- Le THF est distillé sur CaH₂ et la verrerie séchée à l'étuve
- L'acide perfluoré et le carbonyle-diimidazole sont les réactifs commerciaux purs (Fluka).

### c) Montage.

Ballon de 25ml équipé d'une garde desséchante. agitation magnétique avec barreau aimanté et dispositif d'inertage à l'azote ou à l'argon.

### d) Mode opératoire.

Au carbonyl diimidazole en suspension dans le THF, on ajoute l'acide en solution dans le THF ;
le milieu devient homogène et jaunit.

Après agitation une nuit à température ambiante, le THF est évaporé et on obtient une poudre blanche qui ne subit pas de traitement particulier, mais qui doit toutefois être protégée de l'air et de l'humidité.

### 3) Synthèse de la β-cyclodextrine 2,3-perfluorée.

### a) Équation - bilan.

5g TBDMS + 1g NaH puis 19,2g dérivé perfluoré,
soit un léger excès des réactifs (16 équivalents) pour l'introduction de deux motifs perfluorés par unité de sucre dans la cyclodextrine.

### b) Précautions.

- NaH est dégraissé plusieurs fois avec de l'hexane et CH₂Cl₂ est distillé sur CaH₂.

### c) Montage.

- ballon tricol de 250ml avec réfrigérant, garde desséchante et ampoule de coulée. Agitation magnétique avec barreau aimanté, bain marie.

### d) Mode opératoire.

L'hydrure de sodium dégraissé est placé en suspension dans 25ml de dichlorométhane et la βCD PTBDMS en solution dans 25ml de dichlorométhane est alors ajoutée goutte à goutte.

Le milieu réactionnel est ensuite porté à reflux de solvant pendant 30 minutes et le dérivé fluoré, en solution dans le dichlorométhane(25 ml) est alors ajouté (coloration verdatre du mélange).

Le chauffage à reflux de solvant est maintenu pendant une heure (mousses abondantes dans le ballon de réaction).

Après refroidissement le mélange est versé dans l'eau glacée, extrait plusieurs fois avec le chloroforme et séché sur Na₂SO₄.

Le contrôle des opérations est réalisé à l'aide d'analyses du milieu réactionnel (chromatographie couche mince et HPLC). La purification est effectuée sur gel de silice avec une phase mobile et un gradient de solvant CH₂Cl₂ / MeOH / AcOEt.

La pureté du produit final est contrôlée par HPLC (silices greffées C18 , phase inverse methanol-eau) et ses caractéristiques structurales sont établies à l'aide de la spectrométrie de masse (détermination de la masse moléculaire et fractionnement, MALDI TOF et FAB), de l'IRFT (présence du motif carbonylé de l'ester et des chaînes perfluorées essentiellement) et de la RMN 300 ou 500 MHz (rapport d'intégration des H anomères et des signaux des groupements portés par le silicium lié à l'oxygène en position 6, valeurs caractéristiques des carbonyles en positions 2 et 3 dans les spectres ¹³C, mesure des couplages entre noyaux , spectres du fluor).

### Exemple II : Préparation d'un système colloïdal nanovésiculaire dispersible à base de cyclodextrines perfluorées et son utilisation pour vectoriser des principes actifs.

Les dérivés de cyclodextrine selon l'invention permettent de préparer des particules sphériques de type vésiculaire, de taille inférieure à 1000 nm (nanocapsules) et contenant ou pas une molécule active.

### 1) Procédé de préparation de nanocapsules sphériques de type vésiculaire de cyclodextrine perfluorées à 8 carbones.

### Phase 1 :

- béta cyclodextrine perfluorées à 8 carbones : 50 mg
- acétone : 50 ml
- benzoate de benzyle : 2 ml

### Phase 2 :

- Pluronic® F68 : 62,5 mg
- eau déminéralisée ou distillée : 25 ml

La phase 1 est ajoutée sous agitation magnétique à la phase 2. Le milieu devient immédiatement opalescent par formation de nanocapsules de cyclodextrine modifiée. La taille moyenne des nanocapsules mesurée par un diffractomètre à rayon laser

(Nanosizer ® de chez Coultronics) est de 300 nm avec un indice moyen de dispersion de 0,08. La suspension peut être concentrée sous pression réduite au volume désiré, par exemple 5 ml ou plus ou moins.

Après un repos prolongé ( 6 mois ), l'aspect de la suspension de nanocapsules demeure inchangé et on n'observe, en particulier, aucune sédimentation irréversible, ni variation de la taille des nanocapsules .

Ce procédé admet plusieurs variantes comme celles décrites ci-après.

### 2) Procédé de préparation de nanocapsules de cyclodextrine perfluorées à 8 carbones.

On procède comme dans le procédé du point 1, mais en ajoutant la phase aqueuse à la phase acétonique. Les nanocapsules obtenues présentent les mêmes caractéristiques que dans le procédé du point 1.

### 3) Procédé de préparation de nanocapsules de cyclodextrine perfluorées à 8 carbones.

On procède comme dans le procédé du point 1, mais en ajoutant la phase acétonique à la phase aqueuse sans agitation du milieu. Les nanocapsules obtenues ont une taille de 300 nm avec un indice moyen de dispersion de 0,5.

### 4) Procédé de préparation de nanocapsules de cyclodextrine perfluorées à 8 carbones.

On procède comme dans le procédé du point 1, mais sans ajouter d'agent de surface à la phase aqueuse. Les nanocapsules ont une taille moyenne de 500 nm avec un indice moyen de dispersion de 0,6.

### 5) Procédé témoin.

On procède comme décrit dans le procédé du point 1, mais sans la cyclodextrine perfluorée, on constate que 99 % de la quantité d'huile utilisée se sépare au fond du bécher. Cet essai montre le rôle fondamentale de la cyclodextrine modifiée.

### 6) Préparation stérile de nanocapsules de cyclodextrine perfluorée à 8 carbones.

On procède comme dans le procédé du point 1, puis la suspension est stérilisée à l'autoclave à 134°C pendant 15 minutes. La taille moyenne des particules demeure pratiquement inchangée après stérilisation.

### 7) Préparation lyophilisée de nanocapsules de cyclodextrine perfluorée à 8 carbones.

On procède comme dans le procédé du point 1, puis la suspension est lyophilisée. L'addition d'un cryoprotecteur (maltose ou tréhalose) n'est pas indispensable. La taille moyenne des particules demeure inchangée après lyophilisation.

### 8) Stabilité des nanocapsules de cyclodextrine en présence de forces ioniques variables.

On procède comme indiqué dans le procédé du point 1. Après concentration de la suspension de nanocapsules de cyclodextrines jusqu'à un volume de 10 ml, on ajoute progressivement à celle-ci des quantités croissantes de chlorure de sodium. La suspension de nanocapsules est parfaitement stable lorsque la concentration en chlorure de sodium correspond à l'isotonie avec le sang et le demeure jusqu'à une concentration supérieure à 3 fois la concentration isotonique.

### 9) Stabilité des nanocapsules de cyclodextrine en présence d'un milieu acide ou basique.

On procède comme indiqué dans le procédé du point 1. Après concentration de la suspension de nanocapsules de cyclodextrine jusqu'à un volume de 10 ml, on ajoute progressivement à celle-ci des quantités croissantes d'acide chlorhydrique (1N) ou de la soude (1N). La suspension de nanocapsules est parfaitement stable.

### 10) Stabilité des nanocapsules de cyclodextrine à la température.

On procède comme indiqué dans le procédé du point 1. Après concentration de la suspension de nanocapsules de cyclodextrine jusqu'à un volume de 10 ml, on place chaque lot à 4°C, 25°C et 40°C.

Les suspensions demeurent stables dans le temps et ne présentent, après 5 mois de conservation, ni sédimentation irréversible, ni variation de la taille des nanocapsules.

### 11) Préparation de nanocapsules en présence d'un sel.

On procède comme indiqué dans le procédé du point 1, mais la phase aqueuse est additionnée de 90 mg de chlorure de sodium. Après concentration de la suspension de nanocapsules jusqu'à un volume de 10 ml, correspondant compte tenu du chlorure de sodium à l'isotonie avec le sang, les nanocapsules ont une taille moyenne de 320 nm avec un indice moyen de dispersion de 1.

La suspension demeure stable dans le temps et ne présente, après 5 mois de conservation, ni sédimentation irréversible, ni variation de la taille des nanocapsules .

### 12) Addition de non-solvant dans la phase du solvant.

On procède comme indiqué dans le procédé du point 1, la cyclodextrine est dissoute dans un mélange acétone/eau (90/10, v/v), au lieu d'acétone pure. La présence d'une faible proportion de non-solvant de la cyclodextrine dans son solvant, conduit à des nanocapsules dont la taille moyenne est de 380 nm avec un indice moyen de dispersion de 0,5.

### 13) Stabilité des nanocapsules de cyclodextrine aux ultrasons.

On procède comme indiqué dans le procédé du point 1. Après concentration de la suspension de nanocapsules de cyclodextrine jusqu'à un volume de 10 ml, on place la suspension de nanocapsules de cyclodextrine dans un bain à ultrasons pendant trois heures.

La suspension demeure stable dans le temps et ne présente, après 5 mois de conservation, ni sédimentation irréversible, ni variation de la taille des nanocapsules.

### 14) Préparation de nanocapsules en présence d'un principe actif hydrophile.

On procède comme indiqué dans le procédé du point 1, mais on ajoute 10 mg de doxorubicine dans la phase aqueuse. Dans la phase 2 on utilise de l'éthanol et 0,5 ml de benzoate de benzyle. Les nanocapsules obtenues ont une taille moyenne de 300 nm et un indice moyen de dispersion de 1. Après ultracentrifugation et dosage de la doxorubicine dans la phase dispersante, la quantité de principe actif incorporé dans les nanocapsules représente 66 % de la quantité initiale.

### 15) Préparation de nanocapsules en présence d'un principe actif lipophile.

On procède comme indiqué dans le procédé du point 1, mais on ajoute 30 mg d'indométacine et 0,3 ml de benzoate de benzyle dans la phase acétonique. Les nanocapsules obtenues ont une taille moyenne de 320 nm avec un indice de dispersion de 0,5. Après ultracentrifugation et dosage de l'indométacine dans la phase dispersante, la quantité de principe actif incorporé dans les nanocapsules représente 90 % de la quantité initiale.

### 16) Préparation de nanocapsules contenant de la progestérone.

On procède comme indiqué dans le procédé du point 1, mais on ajoute 230 mg de progestérone et 0,6 ml de benzoate de benzyle dans la phase 1. Les nanocapsules obtenues ont une taille moyenne de 120 nm et un indice de dispersion de 0,2. Après ultracentrifugation et dosage de la progestérone dans la phase dispersante, la quantité de principe actif incorporé dans les nanocapsules représente 90% de la quantité initiale.

### 17) Préparation de nanocapsules contenant un colorant lipophile:le soudan III.

On procède comme indiqué dans le procédé du point 1, mais on ajoute 5mg de soudan III dans la phase 1. Une faible quantité est précipitée et reste sur le filtre. Les nanocapsules obtenues ont une taille moyenne de 130 nm et un indice de dispersion de 0,2.

### Exemple III : Préparation d'une suspension colloïdale à base de cyclodextrines perfluorées et son utilisation pour transporter des principes actifs.

### 1) Procédé de préparation de nanosphères sphériques de type matriciel de cyclodextrine perfluorée à 8 carbones.

### Phase 1 :

- bêta cyclodextrine perfluorées à 8 carbones : 50 mg
- acétone : 50 ml

### Phase 2 :

- Pluronic® F68 : 62,5 mg
- eau déminéralisée ou distillée : 25 ml

La phase 1 est ajoutée sous agitation magnétique à la phase 2. Le milieu devient immédiatement opalescent par formation de nanoparticules à base de cyclodextrine modifiée. La taille moyenne des systèmes transporteurs mesurée par un diffractomètre à rayon laser (Nanosizer ® de chez Coultronics) est de 180 nm avec un indice moyen de dispersion de 0,08.

La suspension peut être concentrée sous pression réduite au volume désiré, par exemple 5 ml ou plus ou moins.

Après un repos prolongé (6 mois), l'aspect de la suspension de nanoparticules demeure inchangé et on n'observe, en particulier, aucune sédimentation irréversible, ni variation de la taille des systèmes transporteurs.

Ce procédé admet plusieurs variantes, dont celles décrites ci-après.

### 2) Procédé de préparation de nanosphères de cyclodextrine perfluorées à 8 carbones.

On procède comme dans le procédé du point 1, mais en ajoutant la phase acétonique à la phase aqueuse. Les nanoparticules obtenues présentent les mêmes caractéristiques dans dans le procédé du point 1 ci-dessus.

### 3) Procédé de préparation de nanosphères de cyclodextrine perfluorées à 8 carbones.

On procède comme dans le procédé du point 1, mais en ajoutant la phase acétonique à la phase aqueuse sans agitation du milieu. Les nanoparticules obtenues ont une taille de 200 nm avec un indice moyen de dispersion de 0,5.

### 4) Procédé de préparation de nanosphères de cyclodextrine perfluorées à 8 carbones.

On procède comme dans le procédé du point 1, mais sans ajouter d'agent de surface à la phase aqueuse. Les nanoparticules ont une taille moyenne de 200 nm avec un indice moyen de dispersion de 0,6.

### 5) Préparation stérile de nanosphères à base de cyclodextrine perfluorées à 8 carbones.

On procède comme dans le procédé du point 1, puis la suspension est stérilisée à l'autoclave à 134°C pendant 15 minutes. La taille moyenne des particules demeure pratiquement inchangée après stérilisation.

### 6) Préparation lyophilisée de nanosphères à base de cyclodextrine perfluorées à 8 carbones.

On procède comme dans le procédé du point 1, puis la suspension est lyophilisée. L'addition d'un cryoprotecteur (maltose ou tréhalose) n'est pas indispensable.La taille moyenne des particules demeure inchangée après lyophilisation.

### 7) Stabilité des nanosphères à base de cyclodextrine en présence de forces ioniques variables.

On procède comme dans le procédé du point 1. Après concentration de la suspension de nanosphères jusqu'à un volume de 10 ml, on ajoute progressivement à celle-ci des quantités croissantes de chlorure de sodium. La suspension de nanosphères est parfaitement stable lorsque la concentration en chlorure de sodium correspond à l'isotonie avec le sang et le demeure jusqu'à une concentration supérieure à 3 fois la concentration isotonique.

### 8) Stabilité des nanosphères à base de cyclodextrine en présence d'un milieu acide ou basique.

On procède comme dans le procédé du point 1. Après concentration de la suspension de nanosphères jusqu'à un volume de 10 ml, on ajoute progressivement à celle-ci des quantités croissantes d'acide chlorhydrique (1N) ou de la soude (1N). La suspension de nanosphères est parfaitement stable.

### 9) Stabilité des nanosphères à la température.

On procède comme dans le procédé du point 1. Après concentration de la suspension de nanosphères jusqu'à un volume de 10 ml, on place chaque lot à 4°C, 25°C et 40°C.

Les suspensions demeurent stables dans le temps et ne présentent, après 5 mois de conservation, ni sédimentation irréversible, ni variation de la taille des nanosphères.

### 10) Préparation de nanosphères en présence d'un sel.

On procède comme dans le procédé du point 1, mais la phase aqueuse est additionnée de 90 mg de chlorure de sodium. Après concentration de la suspension de nanosphères jusqu'à un volume de 10 ml, correspondant compte tenu du chlorure de sodium à l'isotonie avec le sang, les nanosphères ont une taille moyenne de 200 nm avec un indice moyen de dispersion de 1.

La suspension demeure stable dans le temps et ne présente, après 5 mois de conservation, ni sédimentation irréversible, ni variation de la taille des nanosphères.

### 11) Addition de non-solvant dans la phase du solvant.

On procède comme dans le procédé du point 1. La cyclodextrine est dissoute dans un mélange acétone/eau (90/10, v/v), au lieu d'acétone pure. La présence d'une faible proportion de non-solvant de la cyclodextrine dans son solvant, conduit à des nanosphères dont la taille moyenne est de 180 nm avec un indice moyen de dispersion de 0,5.

### 12) Stabilité des nanosphères aux ultrasons.

On procède comme dans le procédé du point 1. Après concentration de la suspension de nanosphères jusqu'à un volume de 10 ml, on place la suspension dans un bain à ultrasons pendant trois heures.

La suspension demeure stable dans le temps et ne présente, après 5 mois de conservation, ni sédimentation irréversible, ni variation de la taille des nanosphères.

### 13) Préparation de nanosphères en présence d'un principe actif hydrophile.

On procède comme dans le procédé du point 1, le point 4 ou le 5 ci-dessus, mais en présence de 50 mg d'adénosine -5'- triphosphate (ATP) dissous dans la phase 2.

La taille moyenne des nanosphères est de 175 nm avec un indice moyen de dispersion de 0,08. Le taux d'encapsulation d'ATP, mesuré après ultracentrifugation est voisin de 100% de la quantité mise en oeuvre.

### 14) Préparation de nanosphères en présence d'un principe actif lipophile.

On procède comme dans le procédé du point 1, mais on ajoute 20 mg d'indométacine dans la phase acétonique. Les nanosphères obtenues ont une taille moyenne de 200 nm avec un indice de dispersion de 0,5. Après ultracentrifugation et dosage de l'indométacine dans la phase dispersante, la quantité de principe actif incorporé dans les nanosphères représente 70% de la quantité initiale.

### 15) Préparation de nanosphères contenant de la doxorubicine.

On procède comme dans le procédé du point 1, mais on ajoute 5 mg de doxorubicine dans la phase aqueuse. Les nanosphères obtenues ont une taille moyenne de 200 nm et un indice moyen de dispersion de 1. Après ultracentrifugation et dosage de la doxorubicine dans la phase dispersante, la quantité de principe actif incorporé dans les nanosphères représente 60 % de la quantité initiale.

### 16) Préparation de nanosphères contenant de la progestérone.

On procède comme dans le procédé du point 1, mais on ajoute 150 mg de progestérone dans la phase 1. Les nanosphères obtenues ont une taille moyenne de 120 nm et un indice de dispersion de 0,2. Après ultracentrifugation et dosage de la progestérone dans la phase dispersante, la quantité de principe actif incorporé dans les nanosphères représente 90% de la quantité initiale.

## Revendications

1. Dérivé de cyclodextrine **caractérisé en ce qu'**il est constitué de 6 à 8 résidus cyclodextrinyles identiques ou différents de formule IV suivante : 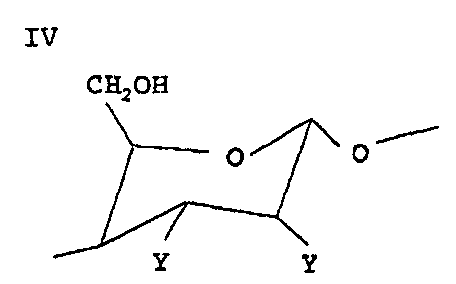 où l'un au moins des groupes Y de l'un au moins des 6 à 8 résidus cyclodextrinyles représente un groupement de formule -(X)-RF, et Y lorsqu'il n'est pas un groupement de formule -(X)-RF est un groupe -OH, où RF est un radical fluoroalkyle de 1 à 20 carbones ou plus dont la chaîne carbonée est éventuellement interrompue par un ou plusieurs hétéroatomes comme l'oxygène ou le soufre, linéaire ou ramifié par un groupe alkyle ou fluoroalkyle, saturée ou non de 1 à 20 carbones ou plus, neutre, zwitterionique, chargée, fonctionnalisée par exemple par un ou plusieurs groupes -COOH, -OH, -CF₃, et X représente un bras de liaison entre un atome de carbone et le groupement de formule RF

2. Dérivé de cyclodextrine selon la revendication 1, **caractérisé en ce que** RF représente de C₃F₇ à C₁₄F₂₉.

3. Dérivé de cyclodextrine selon l'une des revendications 1 ou 2, **caractérisé en ce que** le bras de liaison X est choisi parmi une liaison covalente ou un atome ou un groupe liant l'atome de carbone du résidu cyclodextrinyle et le radical fluoroalkyl RF.

4. Dérivé de cyclodextrine selon l'une des revendications 1 à 3, **caractérisé en ce que** le bras de liaison X est choisi parmi : -NH-, -CO-O-, -O-CO-, -CO-NH-, -NH-CO-, -O-, -O-(CH₂)ₙ-, -(CH₂)ₙ-CO-O-, -O-CO-NH-, -PO₄⁻, -S-.

5. Nanoparticule de taille inférieure à 1000 nm, **caractérisée en ce qu'**elle est constituée :
- d'un ou plusieurs dérivés de cyclodextrine de formule II suivante : 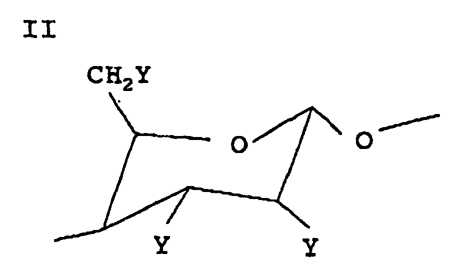 où l'un au moins des groupes Y de l'un au moins des 6 à 8 résidus cyclodextrinyles représente un groupement de formule -(x)-RF, et Y lorsqu'il n'est pas un groupement de formule -(X)-RF est un groupe -OH, où RF et X sont comme définis dans la formule IV ci-dessus,
- et d'une (ou plusieurs) substance(s) active(s),
et **en ce que** ladite (ou lesdites) substance(s) active(s) sont encapsulées dans ladite nanoparticule.

6. Nanoparticule selon la revendication 5, **caractérisée en ce que** la substance active est un principe actif utile en médecine humaine ou animal, un produit de diagnostic.

7. Nanoparticule selon la revendication 5, **caractérisée en ce que** la substance active est un gaz.

8. Nanoparticule selon la revendication 7, **caractérisée en ce que** ledit gaz est l'oxygène.

9. Nanoparticule selon l'une des revendications 5 à 8, **caractérisée en ce qu'**elle est sphérique de type vésiculaire.

10. Un système colloïdal nanovésiculaire dispersible constitué de nanoparticules selon la revendication 9.

11. Nanoparticule selon l'une des revendications 5 à 8, **caractérisée en ce qu'**elle est sphérique de type matriciel.

12. Une suspension colloïdale constituée de nanoparticules selon la revendication 11.

13. Procédé de préparation d'une suspension colloïdale selon la revendication 12, **caractérisé en ce qu'**il comprend les étapes suivants :
a) on prépare une phase liquide constituée essentiellement par une solution de dérivés de cyclodextrine selon l'invention dans un solvant ou mélange de solvants contenant ou non un surfactif et pouvant être additionnée d'une molécule active,
b) on prépare une seconde phase liquide constituée essentiellement par de l'eau ou d'un mélange aqueux, contenant ou non un surfactif et pouvant être additionnée d'une molécule active,
c) on ajoute sous agitation modérée, l'une des phases liquides préparés aux étapes précédentes à l'autre, de manière à obtenir pratiquement instantanément une suspension colloïdale,
d) éventuellement, on élimine tout ou une partie du solvant ou du mélange de solvants et du non-solvant ou du mélange de non-solvants, de manière à obtenir une suspension colloïdale de concentration voulue ou une poudre.

14. Procédé de préparation d'un système colloïdal nanovésiculaire dispersible selon la revendication 10, **caractérisé en ce qu'**il comprend les étapes suivants :
a) on prépare une phase liquide constituée essentiellement par une solution de dérivés de cyclodextrine selon l'une des revendications 1-4 dans un solvant ou mélange de solvants contenant obligatoirement de l'huile, contenant ou non un surfactif et pouvant être additionnée d'une molécule active,
b) on prépare une seconde phase liquide constituée essentiellement par de l'eau ou d'un mélange aqueux, contenant ou non un surfactif et pouvant être additionnée d'une molécule active,
c) on ajoute sous agitation modérée, l'une des phases liquides préparés aux étapes précédentes à l'autre, de manière à obtenir pratiquement instantanément un système colloïdal nanovésiculaire dispersible,
d) éventuellement, on élimine tout ou une partie du solvant ou du mélange de solvants et du non-solvant ou du mélange de non-solvants, de manière à obtenir un système colloïdal nanovésiculaire dispersible de concentration voulue ou une poudre.

## Patentansprüche

1. Derivat von Zyklodextrin **dadurch gekennzeichnet, dass** es aus 6 bis 8 gleichen oder verschiedenen zyklodextrinylen Residuumen der folgenden Formel IV besteht: 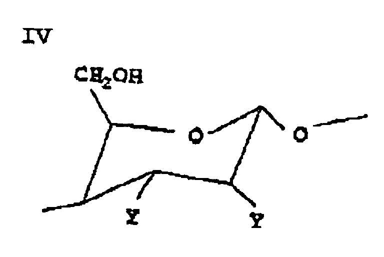 wo mindestens eine der Y Gruppen mindestens eines der 6 bis 8 zyklodextrinilen Residuume eine Gruppierung der Formel -(X)-RF darstellt, und Y wenn es keine Gruppierung der Formel -(X)-RF ist eine -OH Gruppe ist, wo RF ein fluoralkyler Radikal von 1 bis 20 oder mehr Kohlenstoffen ist deren Kohlenstoffkette eventuell durch ein oder mehrere Heteroatome wie Sauerstoff oder Schwefel unterbrochen ist, linear oder verzweigt durch eine Alkylgruppe oder eine Fluoralkylgruppe, gesättigt oder nicht mit 1 bis 20 oder mehr Kohlenstoffen, neutral, zwitterionisch, geladen, zum Beispiel funktionalisiert durch eine oder mehrere -COOH, -OH, -CF₃ Gruppen, und X stellt einen Bindungsarm zwischen einem Kohlenstoffatom und der Gruppierung mit Formel RF dar.

2. Derivat von Zyklodextrin nach Anspruch 1, **dadurch gekennzeichnet, dass** RF von C₃F₇ bis C₁₄F₂₉ darstellt.

3. Derivat von Zyklodextrin nach einem der Ansprüche 1 oder 2, **dadurch gekennzeichnet, dass** der Bindungsarm X ausgewählt ist aus einer kovalenten Bindung oder einem Atom oder einer Gruppe verbindend das Kohlenstoffatom des Zyklodextrinilrestes und den Fluoralkylradikal RF.

4. Derivat von Zyklodextrin nach einem der Ansprüche 1 bis 3, **dadurch gekennzeichnet, dass** der Bindungsarm X ausgewählt ist zwischen: -NH-, -CO-O-, -O-CO-, -CO-NH-, -NH-CO-, -O-, -O-(CH₂)ₙ-, -(CH₂)ₙ-CO-O-, -O-CO-NH-, -PO₄⁻, -S-.

5. Nanopartikel einer Größe kleiner als 1000 nm, **dadurch gekennzeichnet, dass** es besteht aus:
- einem oder mehreren Derivaten von Zyklodextrin der folgenden Formel II: 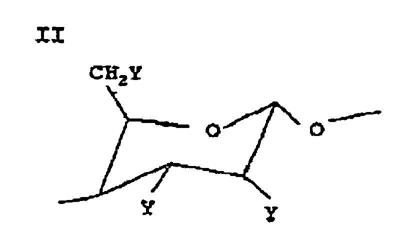 wo mindestens eine der Y Gruppen mindestens eines der 6 bis 8 zyklodextrinilen Residuume eine Gruppierung der Formel -(X)-RF darstellt, und Y wenn es keine Gruppierung der Formel -(X)-RF ist eine -OH Gruppe ist, wo RF und X definiert sind wie in der oben erwähnten Formel IV,
- und einer (oder mehreren) aktive(n) Substanz(en), und indem die besagte (oder besagten) aktive(n) Substanz(en) in besagtem Nanopartikel eingekapselt sind.

6. Nanopartikel nach Anspruch 5, **dadurch gekennzeichnet, dass** die aktive Substanz ein aktiver Grundsatz nützlich in Human- oder Tiermedizin, ein Diagnostikprodukt ist.

7. Nanopartikel nach Anspruch 5, **dadurch gekennzeichnet, dass** die aktive Substanz ein Gas ist.

8. Nanopartikel nach Anspruch 7, **dadurch gekennzeichnet, dass** besagtes Gas Sauerstoff ist.

9. Nanopartikel nach einem der Ansprüche 5 bis 8, **dadurch gekennzeichnet, dass** es kugelförmig von blasenförmigem Typ ist.

10. Ein kolloidales nanoblasenförmiges zerstreubares System bestehend aus Nanopartikeln nach Anspruch 9.

11. Nanopartikel nach einem der Ansprüche 5 bis 8, **dadurch gekennzeichnet, dass** es kugelförmig von matritzenförmigem Typ ist.

12. Eine kolloidale Suspension bestehend aus Nanopartikeln nach Anspruch 11.

13. Verfahren zur Vorbereitung einer kolloidalen Suspension nach Anspruch 12, **dadurch gekennzeichnet, dass** sie folgende Etappen beinhaltet:
a) man bereitet eine flüssige Phase vor bestehend hauptsächlich aus einer Lösung von Derivaten von Zyklodextrin nach der Erfindung in einem Lösungsmittel oder einer Lösungsmittelmischung beinhaltend oder nicht einen Surfaktiv und zusätzlich ein aktives Molekül,
b) man bereitet eine zweite flüssige Phase vor bestehend hauptsächlich aus Wasser oder einer wässerigen Lösung, beinhaltend oder nicht einen Surfaktiv und zusätzlich ein aktives Molekül,
c) man fügt unter mäßigem Schütteln eine der beiden in den vorhergehenden Etappen vorbereiteten flüssigen Phasen zu der zweiten hinzu, auf solche Weise dass man praktisch augenblicklich eine kolloidale Suspension erhält,
d) eventuell eliminiert man das gesamte oder einen Teil des Lösungsmittels oder der Lösungsmittelmischung oder des Nicht-Lösungsmittels oder der Nicht-Lösungsmittelmischung, auf solche Art und Weise dass man eine kolloidale Suspension mit einer gewünschten Konzentration erhält oder ein Puder.

14. Verfahren zur Vorbereitung eines kolloidalen nanoblasenförmigen zerstreubaren Systems nach Anspruch 10, **dadurch gekennzeichnet, dass** sie folgende Etappen beinhaltet:
a) man bereitet eine flüssige Phase vor bestehend hauptsächlich aus einer Lösung von Derivaten von Zyklodextrin nach einem der Ansprüche 1 bis 4 in einem Lösungsmittel oder einer Lösungsmittelmischung zwangsläufig beinhaltend Öl, beinhaltend oder nicht einen Surfaktiv und zusätzlich ein aktives Molekül,
b) man bereitet eine zweite flüssige Phase vor bestehend hauptsächlich aus Wasser oder einer wässerigen Lösung, beinhaltend oder nicht einen Surfaktiv und zusätzlich ein aktives Molekül,
c) man fügt unter mäßigem Schütteln eine der beiden in den vorhergehenden Etappen vorbereiteten flüssigen Phasen zu der zweiten hinzu, auf solche Weise dass man praktisch augenblicklich ein kolloidales nanoblasenförmiges zerstreubares System erhält,
d) eventuell eliminiert man das gesamte oder einen Teil des Lösungsmittels oder der Lösungsmittelmischung oder des Nicht-Lösungsmittels oder der Nicht-Lösungsmittelmischung, auf solche Art und Weise dass man ein kolloidales nanoblasenförmiges zerstreubares System mit einer gewünschten Konzentration erhält oder ein Puder.

## Claims

1. Derivative of cyclodextrin **characterised in that** it is composed of 6 to 8 cyclodextrinyl residues that are identical or different from the following formula IV: 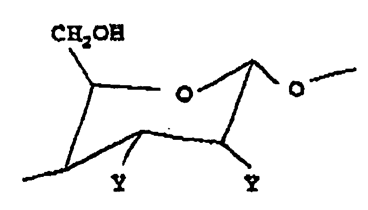 in which at least one of the Y groups in at least one of the 6 to 8 cyclodextrinyl residues represents a group with formula -(X)-RF, and Y when it is not a group with formula -(X)-RF is a -OH group, where RF is a fluoroalkyl radical with 1 to 20 or more carbon atoms in which the carbonaceous chain is possibly interrupted by one or several heteroatoms such as oxygen or sulphur, linear or branched by an alkyl or fluoroalkyl group, saturated or not with 1 to 20 or more carbon atoms, neutral, zwitterionic, charged, functionalised for example by one or several -COOH, -OH, -CF₃ groups, and X represents a linker between a carbon atom and the group with formula RF.

2. Derivative of cyclodextrin according to claim 1, **characterised in that** RF represents from C₃F₇ to C₁₄F₂₉.

3. Derivative of cyclodextrin according to either claim 1 or 2, **characterised in that** the linker X is chosen from among a covalent bond or an atom or a group bonding the carbon atom of the cyclodextrinyl residue and the RF fluoroalkyl radical.

4. Derivative of cyclodextrin according to any one of claims 1 to 3, **characterised in that** the linker X is chosen from among -NH-, -CO-O-, -O-CO-, -CO-NH-, -NH-CO-, -O-, -O- (CH₂)ₙ-, -(CH₂)ₙ-CO-O, -O-CO-NH-, -PO₄-, -S-.

5. Nanoparticle smaller than 1000 nm, **characterised in that** it is composed of:
- one or several derivatives of cyclodextrin with the following formula II: 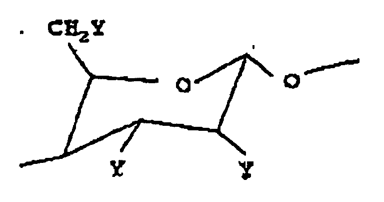 in which at least one of the groups Y in at least one of the 6 to 8 cyclodextrinyl residues represents a group with formula -(X)-RF, and Y when it is not a group with formula - (X)-RF, is a -OH group, where RF and X are as defined in the formula IV above,
- and one (or more) active substance(s),
and **in that** the said active substance(s) is (are) encapsulated in the said nanoparticle.

6. Nanoparticle according to claim 5, **characterised in that** the active substance is an active constituent useful in human or animal medicine, or a diagnostic product.

7. Nanoparticle according to claim 5, **characterized in that** the active substance is a gas.

8. Nanoparticle according to claim 7, **characterized in that** said gas is oxygen.

9. Nanoparticle according to any one of claims 5 to 8, **characterized in that** it is a vesicular type sphere.

10. A dispersible nanovesicular colloidal system composed of nanoparticles according to claim 9.

11. Nanoparticle according to any one of claims 5 to 8, **characterized in that** it is a matrix type sphere.

12. A colloidal suspension composed of nanoparticles according to claim 11.

13. Method of preparing a colloidal suspension according to claim 12, **characterized in that** it comprises the following steps:
a) a liquid phase is prepared composed essentially of a solution of cyclodextrin derivatives according to the invention in a solvent or solvent mix that may or may not contain a surfactant and to which an active molecule can be added,
b) a second liquid phase is prepared, composed essentially of water or an aqueous mixture, containing or not containing a surfactant and to which an active molecule can be added,
c) one of the liquid phases prepared in the previous steps is added to the other phase, while stirring moderately, so as to obtain a colloidal suspension almost instantaneously,
d) possibly, all or part of the solvent or solvent mix and the non-solvent or non-solvent mix is eliminated, so as to obtain a colloidal suspension with a required concentration or a powder.

14. Method for preparation of a dispersible nanovesicular colloidal system according to claim 10, **characterized in that** it comprises the following steps:
a) a liquid phase is prepared composed essentially of a solution of cyclodextrin derivatives according to one of claims 1 to 4 in a solvent or solvent mix that necessarily contains oil, that may or may not contain a surfactant and to which an active molecule can be added,
b) a second liquid phase is prepared, composed essentially of water or an aqueous mixture, containing or not containing a surfactant and to which an active molecule can be added.
c) one of the liquid phases prepared in the previous steps is added to the other phase, while stirring moderately, so as to obtain a colloidal suspension almost instantaneously,
d) possibly, all or part of the solvent or solvent mix and the non-solvent or non-solvent mix is eliminated, so as to obtain a nanovesicular colloidal system with a required concentration or a powder.
